# EUROPÄISCHE PATENTSCHRIFT

(11) **EP 0 757 055 B1**
(45) Veröffentlichungstag und Bekanntmachung des Hinweises auf die Patenterteilung: **27.10.1999**
(21) Anmeldenummer: 96110132.6
(22) Anmeldetag: 23.06.1996
(51) Int. Cl.: C07H 17/08, A61K 31/70, C07C 62/32, C07C 62/34, C07C 69/757, A61K 31/19

(54) **Glykoside, deren zuckerfreie Abbauprodukte und Derivate derselben**
Glycosides, sugar-free degradation products and derivatives of the same
Glycosides, produits de dégradation exempt de sucre, et dérivés de tels produits

(30) Priorität: 30.06.1995 CH 193095
(43) Veröffentlichungstag der Anmeldung: 05.02.1997
(73) Patentinhaber: Cerbios-Pharma S.A., CH-6917 Barbengo (CH)
(72) Erfinder: Jaton, Jean-Claude, 1226 Thonex (CH); Marazza, Fabrizio, 6986 Novaggio (CH); Lewenstein, Ari, 8002 Zürich (CH); Sirotnak, Francis M., New York 10028 (US); Jaun, Bernhard, 8117 Fällanden (CH)
(74) Vertreter: Blum, Rudolf Emil Ernst

(56) Entgegenhaltungen:
- EP-A- 0 220 453
- WO-A-93/08799
- DE-A- 3 612 278
- S.CHANDRA LAHIRI ET AL.: "Synthesis and Pharmacology of Some Pyrroles and Indan Amines : Hexahydro Indeno (1,2-c) Pyrroles and Indan Amines." JOURNAL OF PHARMACEUTICAL SCIENCES, Bd. 57, Nr. 6, 1968, Seiten 1013-1016, XP002056178

## Beschreibung

Die vorliegende Erfindung betrifft neue Glykoside, die aus dem wässrigen Extrakt von Pflanzenpollen isoliert wurden und ferner neue Aglykone, die nach der Abspaltung von Zuckerbetandteilen aus diesen Glykosiden erhalten wurden und Derivate der neuen Aglykone.

Die genannten neuen Verbindungen können beispielsweise als Wirkstoffe in pharmazeutischen Präparaten eingesetzt werden.

### BESCHREIBUNG DES STANDES DER TECHNIK

Verfahren zur Herstellung von Extrakten aus Pflanzenpollen sind seit langem bekannt, und es sei in diesem Zusammenmhang beispielsweise auf die deutsche Patentschrift Nr. 1 467 750 und die österreichische Patentschrift Nr. 255 634 verwiesen, in denen Verfahren zur Herstellung von solchen Pollenextrakten beschrieben sind, die von den hochmolekularen Proteinen der Pollenhülle im wesentlichen befreit sind, welche möglicherweise Allergien hervorrufen können. Die entsprechenden, mit wässrigen Extraktionsmitteln, bzw. mit nicht wässrigen Extraktionsmitteln gewonnenen Pollenextrakte und auch Mischungen derselben, sind seit vielen Jahren im Handel erhältlich, und sie werden als Stärkungsmittel zur Erhöhung der Abwehrkräfte, zur Behandlung von Prostataleiden, zur Beschleunigung der Heilung von Wunden und Knochenbrüchen und als entzündungshemmende Mittel eingesetzt.

Ferner werden in der europäischen Patentschrift 201 053 Verfahren zur Herstellung eines pharmazeutischen Präparates zur prophylaktischen Behandlung von Allergien beschrieben, das als Wirkstoff einen mit wässrigen Extraktionsmitteln oder mit nicht wässrigen Extraktionsmitteln gewonnenen entsprechenden Pollenextrakt enthält.

Des weiteren wurde auch bereits versucht, aus den von hochmolekularen Proteinen befreiten, mit wässrigen Extraktionsmitteln gewonnenen Pollenextrakte Fraktionen zu isolieren, die an Wirkstoffen angereichert sind. In der europäischen Patentschrift 220 453 und in der entsprechenden US Patentschrift 4 952 399, werden die von hochmolekularen Proteinen befreiten, mit wässrigen Extraktionsmitteln gewonnenen Pollenextrakte zur Herstellung von pharmazeutischen Präparaten eingesetzt, welche das Wachstum von Tumorzellen hemmen. Gemäss diesen Patentschriften werden als Wirkstoff auch solche Fraktionen des Pollenextraktes eingesetzt, die an Aminosäuren und Peptiden und/oder proteinartigen Substanzen angereichert sind, bzw. Fraktionen, die ein Molekulargewicht von weniger als 750, beispielsweise im Bereich von 650-750, aufweisen.

Die dort beschriebenen Pollenextrakte, bzw. die aus den Pollenenextrakten isolierten Wirkstoff-Fraktionen, weisen also eine cytostatische Wirksamkeit auf, d.h. das Wachstum von verschiedenen Krebszellen, wie z.B. Leukämiezellen und menschliche Prostatakrebszellen, Kehlkopfkrebszellen, Leberkrebszellen, Blasenkrebszellen und Brustkrebszellen, konnte in entsprechenden Zellkulturen stark gehemmt werden.

Weitere Arbeiten, die mit den wässrigen Extrakten von Pflanzenpollen durchgeführt wurden, führten jetzt überraschenderweise zur Isolierung von proteinfreien Wirkstoff-Fraktionen, bzw. Wirkstoffen, welche Glycoside sind, bzw. Aglycone darstellen, die durch hydrolytische Spaltung der glycosidischen Wirkstoff-Fraktionen erhalten wurden.

Ueberraschenderweise zeigte es sich ferner, dass diese neuen Wirkstoff-Fraktionen, bzw. die durch deren Spaltung erhaltenen Aglycone sich bezüglich ihrer pharmazeutischen Wirksamkeit von den früher aus Pollen isolierten proteinhaltigen Wirkstoff-Fraktionen unterscheiden.

Die neuen Wirstoff-Fraktionen, bzw. die daraus isolierten Wirkstoffe, bzw. synthetisch hergestellte entsprechende neue Wirkstoffderivate, weisen nämlich die Eigenschaft auf, die Immunreaktion warmblütiger Tiere zu modulieren.

### BESCHREIBUNG DER ERFINDUNG

Aus dem wässrigen Extrakt von Pflanzenpollen wurden jetzt neue Glycoside isoliert, welche die folgende Formel I 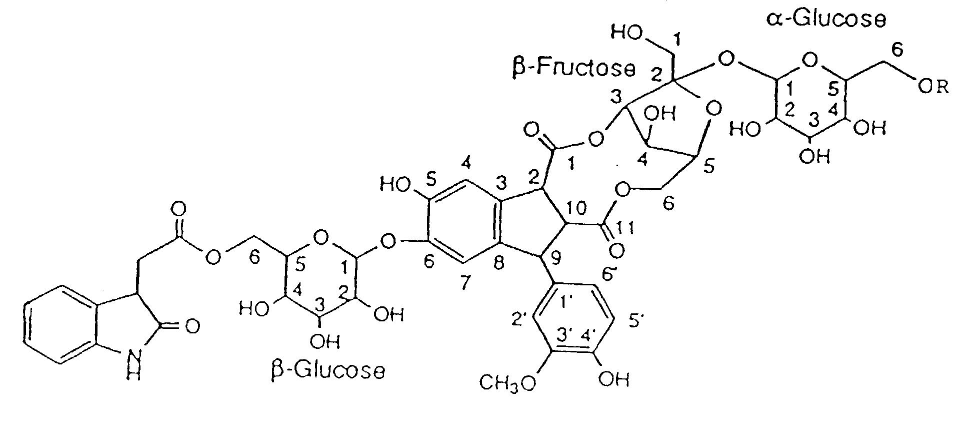 aufweisen.

In dieser Formel I bedeutet
R ein Wasserstoffatom oder einen weiteren Zuckerrest, insbesondere Glucoserest, der glycosidisch an die hier dargestellte Glucose in der 6-Stellung derselben gebunden ist.

In den Glycosiden der oben angegebenen Strukturformel I ist also der Rest einer unsubstituierten 2-Oxindol-3-essigsäure der Formel A 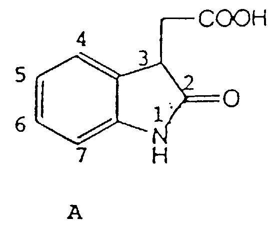 über seine Carboxylgruppe esterartig an die Hydroxylgruppe in der 6-Stellung eines Moleküls der Glucose gebunden.

Dieses Glucosemolekül ist seinerseits β-glycosidisch an die phenolische Hydroxylgruppe in der 6-Stellung des Indan-Gerüstes eines Aglycons gebunden, welches die folgende Formel II 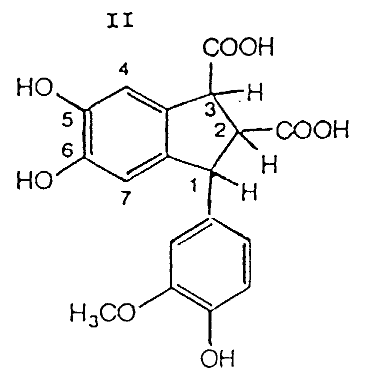 aufweist. In dieser Verbindung der Formel II ist also in der 1-Stellung des Indan-Gerüstes ein Phenylkern gebunden, der in der p-Stellung mit einer Hydroxygruppe und in der m-Stellung mit einer Methoxygruppe substituiert ist. Des weiteren befinden sich in dieser Verbindung der Formel II die an die Stellungen 2 und 3 gebundenen Carboxylgruppen in cis-Stellung zueinander, während sich der an die 1-Stellung des Indangerüstes gebundene Phenylkern in trans-Stellung zu der Carboxylgruppe befindet, die an die 2-Stellung des Indangerüstes gebunden ist.

Sowohl das Indan-Derivat der angegebenen Formel II als auch die übrigen hier nicht dargestellten möglichen sterischen Konfigurationen bezüglich der Stellung der Wasserstoffatome und der Substituenten in den Stellungen 1, 2 und 3 sind neue chemische Verbindungen.

Ein weiterer Gegenstand der vorliegenden Erfindung sind also diese neuen chemischen Verbindungen der oben angegebenen Formel II und deren verschiedenen steroisomeren Formen.

In dem Glycosid der Formel I sind die beiden Carbonsäuregruppierungen der Dicarbonsäure der Formel II esterartig an die Hydroxylgruppen in den Stellungen 3 und 6 des Fructoserestes gebunden, und zwar unter Ausbildung eines 10-gliedrigen Dilactonringes.

In denjenigen Glycosiden der Formel I, in welchen R ein Wasserstoffatom bedeutet, ist ferner an die β-Fructose glykosidisch ein Rest der α-Glucose gebunden, sodass die an die beiden Carboxylgruppen der Verbindungen der Formel II gebundene Struktur aus zwei Hexoseeinheiten derjenigen der Saccharose entspricht.

In denjenigen Verbindungen der Formel II, in welchen R einen weiteren glycosidisch gebundenen Hexoserest darstellt, ist dieser Hexoserest vorzugsweise der Rest einer weiteren Glucoseeinheit.

Aus der Indan-Verbindung der Formel II sind synthetisch weitere Derivate der folgenden Formel III 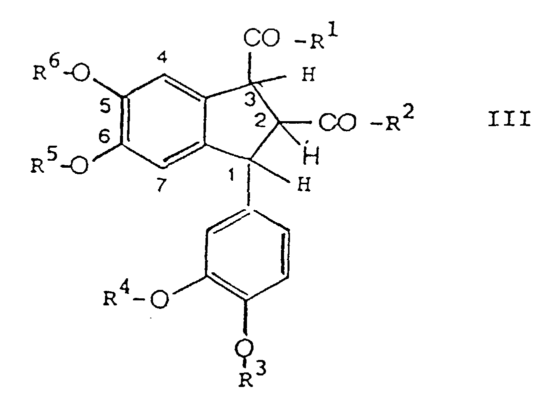 erhältlich.

In diesen Derivaten bedeuten
R¹ und R² unabhängig voneinander Gruppen der Formel

-O-X,

in welchen
X für Wasserstoff, substituierte oder unsubstituierte Alkylgruppen mit 1-6 Kohlenstoffatomen, beispielsweise Aralkylgruppen oder substituierte oder unsubstituierte Arylgruppen oder ein oder mehrere esterartig gebundene Hexosereste stehen oder R¹ und R² gemeinsam eine zweiwertige Gruppierung der Formel

   -O- oder -O-Y-O-

   bedeutet, wobei in diesen Formeln
Y für einen zweiwertigen organischen Rest, insbesondere einen aliphatischen, cycloaliphatischen oder aromatischen Kohlenwasserstoffrest steht und R³, R⁴, R⁵ und R⁶ unabhängig voneinander für Wasserstoffatome, gegebenenfalls substituierte Alkylreste, gegebenenfalls substituierte Cycloalkylreste, gegebenenfalls substituierte Arylreste oder Acylreste von aliphatischen, cyclo-aliphatischen oder aromatischen Carbonsäuren oder ein oder mehrere Hexosereste stehen.

Wie bereits erwähnt, wurden die neuen Glycoside der Formel I aus wässrigen Extrakten von Pflanzenpollen verschiedenster Arten isoliert.

Das Ausgangsmaterial, aus welchem diejenige Fraktion isoliert wird, welche diese Glycoside enthält, ist ein mit einem wässrigen Extraktionsmittel aus Pflanzenpollen gewonnener Extrakt, der höchstens 5 Gew.-% an hochmolekularen Proteinen enthält oder frei von diesen ist. Die Herstellung dieser von hochmolekularen Proteinen befreiten Pflanzenpollenextrakte erfolgt nach demjenigen Verfahren, das im Anspruch 5 des europäischen Patentes 220 453, Dokument B, erläutert wird.

Während in dem oben genannten europäischen Patent Fällungsverfahren, bzw. säulenchromatographische Trennungen durchgeführt wurden, um aus dem genannten Pollenextrakt eine an Aminosäuren, Proteinen und/oder proteinartigen Substanzen angereicherte Fraktion zu isolieren, insbesondere eine Fraktion, deren Molekulargewicht vorzugsweise im Bereich von 650-750 liegt, werden jetzt andere Isolationsverfahren durchgeführt, um solche Fraktionen zu isolieren, die frei von Aminosäuren, Proteinen und/oder proteinartigen Substanzen sind, und die Glycoside der Formel I enthalten.

Ein weiterer Gegenstand der vorliegenden Erfindung ist ein Verfahren zur Isolierung von Fraktionen, die Glycoside enthalten, aus dem mit wässrigen Extraktionsmitteln gewonnenen Pollenextrakt und dieses Verfahren ist dadurch gekennzeichnet, dass man den Pollenextrakt, der als weitere Bestandteile auch noch Maltodextrine enthalten kann, in Wasser löst und einer Dialyse mit einem Membran unterwirft, die Substanzen eines Molekulargewichtes von über 1000 Dalton zurückhält, wobei das durch die Membran hindurch diffundierende Material verworfen wird und
in einem zweiten Dialyseschritt dasjenige Material, das nicht durch die Membran hindurchdiffundierte, einer Dialyse unter Verwendung einer Membran unterworfen wird, die Materialien mit einem Molekulargewicht über 3500 Dalton zurückhält, vorzugsweise einer Membran, die Materialien mit einem Molekulargewicht über 2000 Dalton zurückhält, und dann das durch diese Membran hindurchtretende Dialysat unter Vakuum eindampft und daraus durch Gelfiltration Fraktionen isoliert, welche die Glycoside der Formel I enthalten.

Gemäss einer Ausführungsart dieses Isolierungsverfahrens wird der als gelblich-weisses Pulver gewonnene Pollenextrakt zunächst in etwa der 3-4-fachen Menge an destilliertem Wasser gelöst und diese Lösung unter Verwendung von Membranen, die Substanzen mit einem Molekulargewicht über 1000 Dalton zurückhalten, gegen Wasser in der Kälte dialysiert. Beispielsweise lässt sich diese Dialyse unter Verwendung von Schläuchen aus der molekularporösen Membran durchführen, und es ist vorteilhaft, nach etwa 24 Stunden das hindurchdiffundierte Material zu verwerfen und als Aussenlösung eine frische Lösung an destilliertem Wasser einzusetzen und nochmals 24 Stunden dialysiert.

Nach diesem Dialyseschritt wird die Innenlösung, also das Material, das durch die Membran nicht hindurchgetreten ist, konzentriert, im allgemeinen auf ein Volumen, das etwa die Hälfte des Volumens der wässrigen Lösung beträgt, die dieser Dialyse unterworfen wurde. Diese konzentrierte Lösung des von der Membran zurückgehaltenen Produktes weist eine braune Farbe auf, und beim Eindampfen zur Trockene zeigt es sich, dass die Ausbeute 12 bis 25%, bezogen auf das gelblich-weisse Pulver, das als Ausgangsmaterial eingesetzt wird, beträgt.

In einem zweiten Dialyseschritt wird das nach dem Lyophilisieren erhaltene Produkt des ersten Dialyseschrittes in der 2-5-fachen Menge an Wasser gelöst, und die wässrige Lösung wird unter Verwendung einer Membran, die Produkte eines Molekulargewichts von über 2000 Dalton nicht passieren lässt, gegen Wasser dialysiert. Die Dialyse erfolgt während einer Woche, wobei etwa das 20-fache Volumen an Wasser als Aussenlösung eingesetzt wird und diese Aussenlösung täglich gewechselt wird.

Die Aussenlösungen werden miteinander vereinigt und konzentriert und anschliessend unter Vakuum zur Trockene eingedampft. Die Ausbeute beträgt 10-30%, bezogen auf das diesem zweiten Dialyseschritt unterworfene feste Ausgangsmaterial.

Das Material, das durch diese Membran mit einer Ausschlussgrenze von 2000 Dalton nach einer Woche nicht hindurchdiffundiert ist, also die Innenlösung, wird verworfen.

Das bei dieser zweiten Dialyse gewonnene Produkt wird als A2 bezeichnet.

Aus diesem Produkt A2 werden durch Gelfiltration drei aktive Fraktionen isoliert, die mit IIIa, IIIb und IIIc bezeichnet werden.

Die Fraktion IIIb wird durch eine weitere, ähnlich durchgeführte Gelfiltration in fünf Fraktionen aufgeteilt, von denen die Fraktion III-b5 diejenige ist, die in den durchgeführten pharmakoligischen Tests die höchste Aktivität aufweist.

Diese Fraktion III-b5 wird durch Hochdruckflüssigchromatographie weiter gereinigt, wobei zwei Hauptfraktionen isoliert werden, die mit III-b51 und III-b52, oder abgekürzt als b51 oder b52 bezeichnet werden.

Es zeigte sich, dass die Fraktion b51 und auch die Fraktion b52, ein Molekulargewicht von 1001 Dalton aufweist und dass die Massenspektren von b51 und b52 praktisch identisch sind.

Des weiteren zeigte es sich, dass sich in wässriger Lösung b51 in b52 umwandelt und vice versa, wobei nach der Umwandlung, unabhängig davon, ob man von b51 oder von b52 ausgeht, das Mengenverhältnis etwa 60 Gew.-% b51 und etwa 40 Gew.-% b52 beträgt.

Durch weitere analytische Untersuchungen mit kernmagnetischen Resonanzspektren konnte ferner gezeigt werden, dass sowohl das Glycosid b51 als auch das Glycosid b52 eine solche Verbindung der Formel I ist, in welcher der Rest R für Wasserstoff steht.

Obwohl dieses Glycosid der Formel I eine grosse Anzahl an chiralen Zentren aufweist, nämlich beispielsweise die Kohlenstoffatome in den Stellungen 2, 10 und 9 des Indan-Gerüstes, so wird dennoch der Unterschied zwischen dem Glycosid b51 und dem Glycosid b52, lediglich durch eine unterschiedliche Konfiguration am chiralen Zentrum der Indolylessigsäure verursacht. Entsprechende Untersuchungen haben gezeigt, dass sich das Glycosid b51 von dem Glycosid b52 bezüglich der Konfiguration am chiralen Zentrum des Kohlenstoffatoms in der 3-Stellung der 2-Oxindol-3-essigsäure unterscheidet.

Wenn man das Glycosid der Formel I, in welcher R ein Wasserstoffatom ist, unter stark sauren Bedingungen hydrolysiert, dann wird durch Umlagerung aus der 2-Oxindol-3-essigsäure der Formel A die 2-Oxo-1,2,3,4-tetrahydrochinolin-4-carbonsäure gebildet und auch die Indan-dicarbonsäure der Formel II wird als Aglycon von den Zuckerresten abgespaltet.

Die 2-Oxindol-3-essigsäure der Formel A, wie auch die 2-Oxo-1,2,3,4-tetrahydrochinolin-4-carbonsäure sind in Form ihres Racemates seit langem bekannt. Das Indan-Derivat der Formel II stellt jedoch, wie bereits erwähnt wurde, eine neue chemische Verbindung dar.

Das Indan-Derivat der Formel II weist ein Molekulargewicht von 360 Dalton auf. Diese Dicarbonsäure spaltet jedoch aufgrund der cis-Stellung der beiden Carboxylgruppen sehr leicht Wasser ab, sodass im Massenspektrum, je nach Aufnahmebedingungen, praktisch nur ein dem Anhydrid entsprechender Massenpeak beobachtet wurde, der ein um 18 Dalton geringeres Molekulargewicht aufweist, also ein Molekulargewicht von 342 Dalton.

Des weiteren lassen sich aus der Fraktion IIIa durch Säulenchromatographie zwei Haupt-peaks isolieren, die als A3 und A4 bezeichnet werden. Von diesen ergibt der Peak A3 bei der Hochdruckflüssigchromatographie drei Hauptfraktionen, die als A31, A32 und A34 bezeichnet werden.

Aus dem zweiten mit A4 bezeichneten Peak lassen sich durch Hochdruckflüssigchromatographie zwei Fraktionen isolieren, die als A41 und A42 bezeichnet werden. Jede dieser beiden Fraktionen weist ein Molekulargewicht von 1163 Dalton auf, und es zeigte sich, dass in einer wässrigen Lösung wiederum ein Gleichgewicht zwischen A41 und A42 besteht.

Bei der sauren Hydrolyse ergibt das Paar A41/A42 unter anderem ein Aglycon, das mit demjenigen Aglycon identisch sind, das durch die saure Hydrolyse aus dem Paar b51/b52 erhalten wird.

Durch weitere Untersuchungen wurde bewiesen, dass das Paar A41/A42 dasjenige Glycosid der Formel I ist, in welchem R für einen weiteren, an den Glucoserest des Saccharoseteils glycosidisch gebundenen Glucoserest steht.

Wenn man nämlich das Paar A41 A42 mit einer α-Glucosidase behandelt, dann wird aus der Verbindung der Formel I der Glucoserest R abgespaltet, und man erhält das Paar b51/b52, also diejenige Verbindung der Formel I, in welcher R ein Wasserstoffatom ist.

In gleicher Weise, wie dies weiter vorne für das Glycosid b51 und das Glycosid b52 erläutert wurde, unterscheidet sich das Glycosid A41 von dem Glycosid A42 lediglich bezüglich der Konfiguration an demjenigen chiralen Zentrum, welches durch das Kohlenstoffatom in der 3-Stellung des heterocyclischen Ringes des Restes der 2-Oxindol-3-essigsäure gebildet wird.

Während, wie bereits erwähnt wurde, die Verbindung der Formel I, in welcher R ein Wasserstoffatom ist und auch die Verbindung der Formel I, in welcher R für den α-glycosidisch gebundenen Glucoserest steht, bei der sauren Hydrolyse, beispielsweise unter Verwendung 1-normaler Salzsäure bei 100° das Aglycon der Formel II und auch das Aglycon, welches die aus der 2-Oxindol-3-essigsäure der Formel A gebildete 2-Oxo-1,2,3,4-tetrahydrochinolin-4-carbonsäure ist, liefert, so gelingt es unter milden basischen Bedingungen lediglich den an den Glucoserest gebundenen Rest der 2-Oxindol-essigsäure abzuspalten, und man erhält so eine neues Glycosid, welches der folgenden Formel IV 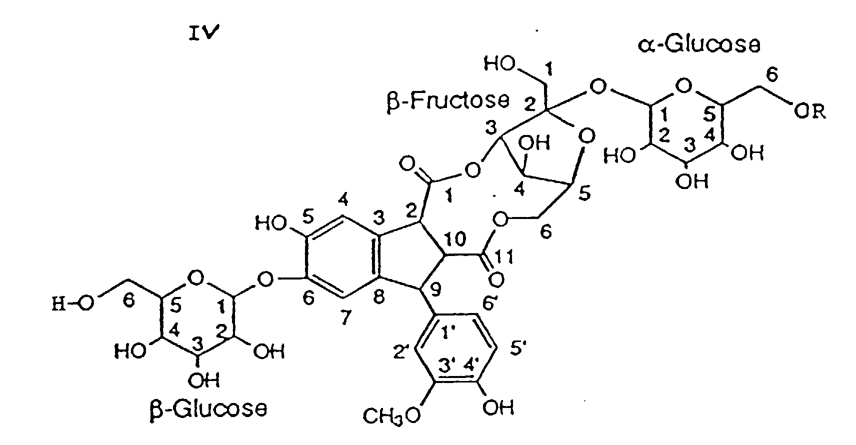 entspricht, wobei in dieser Formel R entweder für ein Wasserstoffatom oder für einen Hexoserest steht, der identisch mit dem Hexoserest des Ausgangsmateriales ist. Dementsprechend ist ein Rest R in der Bedeutung eines Hexoserestes vorzugsweise ein α-glucosidisch gebundener Glucosrest.

Aus diesem Glycosid können durch weitere Behandlungen noch Glucosereste abgespaltet werden, beispielsweise ein Rest R in der Bedeutung eines α-glucosidisch gebundenen Glucoserestes durch Behandlung mit α-Glucosidase abgespaltet werden, sodass man entsprechende Produkte der oben angegebenen Formel erhält, in welchen R für Wasserstoff steht.

Des weiteren ist es auch möglich, in beliebiger Reihenfolge den an die phenolische OH-Gruppe gebundenen Glucoserest abzuspalten und ferner den esterartig an die beiden Dicarbonsäurereste gebundenen Rest der β-Fructose abzuspalten, sodass man schliesslich aus dem Glycosid der oben angegenen Formel durch stufenweise Abspaltung von Zuckerresten, bzw. Saccharose, das Aglycon der weiter vorne angegebenen Formel II erhält.

Die neuen Glycoside der Formel I und der oben angegebenen Formel IV, sowie das Aglycon der Formel II und die aus diesem hergestellten Derivate der Formel III weisen interessante pharmakologische Wirksamkeiten auf. So sind diese neuen Verbindungen beispielsweise in der Lage, eine Modulierung des Immunsystems warmblütiger Tiere hervorzurufen.

Ein weiterer Gegenstand der vorliegenden Erfindung sind pharmazeutische Präparate zur Modulierung des Immunsystems bei warmblütigen Tieren, die dadurch gekennzeichnet sind, dass sie als Wirkstoff ein Glycosid der weiter vorne angegebenen Formeln I oder IV oder ein Aglycon der weiter vorne angegebenen Formel II oder ein Derivat des Aglycons der weiter vorne angegebenen Formel III enthalten.

Weitere Tests zeigten, dass die Wirksamkeit der erfindungsgemässen neuen Glycoside, Aglycone und deren Derivate gehemmt wird, wenn zusätzlich solche Faktoren anwesend sind, die zu einer Hemmung der Stimulierung der Makrophagen oder zu einer Hemmung der Stimulierung der T-Zellen führen.

Die neuen Glycoside der Formeln I und IV, sowie das Algycon der Formel II und deren Derivate der Formel IV, können als Wirkstoffe in solchen pharmazeutischen Präparaten eingesetzt werden, durch welche Tumore, verschiedene Viruskrankheiten, insbesondere solche, die durch Retroviren hervorgerufen werden, wie zum Beispiel Aids, und andere krankhafte Zustände behandelt werden können, die mit einer Schwächung des Immunsystems, bzw. mit einer reduzierten Immunreaktion verbunden sind.

Durch entsprechende Tests konnte gezeigt werden, dass die Lebensdauer von Mäusen, denen verschiedene Krebszellen eingepflanzt worden waren, durch die Verabreichung der Glycoside der Formeln I und IV und durch die Verabreichung des Aglycons und dessen Derivate wesentlich verlängert werden, und zwar im Vergleich zu einer Vergleichsgruppe, an die keine derartigen neuen Verbindungen verabreicht worden war. Besonders gute Effekte konnten mit eingepflanztem Lungenkarzinom (Lewis Lung), Sarkom (S180) und Brustkarzinom (E0771) nachgewiesen werden und auch eine gewisse Aktivität bei Fibrosarkom (T241) und Dickdarmkarzinom (C38) festgestellt werden. Durch die Verabreichung dieser Verbindungen konnten sogar Krebs tumoren bei Mäusen, die nach der Einpflanzung entsprechender Krebszellen entstanden waren, vollständig ausgeheilt werden.

In den nachfolgenden Beispielen werden spezielle Ausführungsarten des Verfahrens zur Isolierung der neuen Glycoside der Formel I aus dem wässrigen Extrakt von Pollen beschrieben.

Des weiteren werden auch Verfahren zur Herstellung von zuckerfreien Abbauprodukten aus diesen Glycosiden erläutert.

### Beispiel 1

Isolierung von solchen Fraktionen aus Pollenextrakten, die an Glycosiden der Formel I angereichert sind.

Aus den Pollen von Pflanzen, beispielsweise Roggen, wurde durch Extraktion mit einem Gemisch aus Wasser und einem vollständig wasserlöslichen organischen Lösungsmittel, beispielsweise Aceton, ein wässriger Pollenextrakt hergestellt. Zu diesem Zwecke rührte man die Pollen bei einer Temperatur von 30-32^{o} C während 48 Stunden. Dabei werden durch eine Autofermentation die hochmolekularen Proteine und Zucker des Pollens abgebaut. Es wurde dann noch während weiterer 20 bis 30 Stunden bei 30^{o} gerührt.

Das wässrige Medium wurde von den Pollen abfiltriert, und die so erhaltene klare Lösung unter Vakuum zur Trockene eingedampft. Man erhielt dabei den getrockneten Pflanzenpollenextrakt in Form eines gelblich-weissen Pulvers.

### Durchführung des ersten Dialyseschrittes

80 g des so erhaltenen gelblich-weissen Pulvers wurde in 280 ml destilliertem Wasser gelöst. Diese Lösung wurde in einen Schlauch aus einer molekular porösen Membran gefüllt, die Substanzen eines Molekulargewichts von weniger als 1000 Dalton hindurchtreten liess.

Die Länge des Schlauches betrug etwa 45 cm, und er wurde zu etwa einem Drittel seines Gesamtvolumens mit dieser Lösung gefüllt. Sechs derartige Schläuche wurden hergestellt und in 10 l kaltes Wasser eingebracht und die Dialyse in der Kälte (etwa 4^{o} C) während 24 Stunden durchgeführt. Nach dieser Zeit wurde die Aussenlösung entfernt und verworfen und gegen 10 l kaltes Wasser ausgetauscht. Nach einer weiteren Dialysezeit von 48 Stunden wurde das in den Schläuchen verbliebene Material entnommen und der Inhalt jedes der sechs Schläuche unter Vakuum zur Trockene eingedampft.

Man erhielt dabei 20 g eines Trockenrückstandes, entsprechend einer Ausbeute von 25% der theoretischen Ausbeute, bezogen auf das eingesetzte Ausgangsmaterial.

### Durchführung des zweiten Dialyseschrittes

Dieser zweite Dialyseschritt wurde unter Verwendung einer Membran durchgeführt, die Substanzen eines Molekulargewichts von 2000 Dalton zurückhielt.

20 g des aus dem ersten Dialyseschritt erhaltenen Trockenproduktes wurden in 100 ml Wasser gelöst, und die Lösung in vier Dialyseschläuche gefüllt, von denen jeder etwa zu einem Drittel seines Volumens gefüllt war.

Diese vier Schläuche wurden gegen 2 l Wasser während eines Tages dialysiert, und dann wurde die Aussenlösung gewonnen und die Dialyse gegen 2 l frisches Wasser weitergeführt. Insgesamt wurde unter täglichem Wechsel der Aussenlösung die Dialyse während einer Woche durchgeführt.

Anschliessend wurden die Aussenlösungen miteinander vereinigt und unter Vakuum zunächst auf 100 ml konzentriert und dann zur Trockene eingedampft.

Nach dieser einwöchigen Dialyse wurde der verbliebene Schlauchinhalt, also die Innenlösung, verworfen.

Man erhielt bei diesem zweiten Dialyseschritt 5-6 g eines leicht gelblich gefärbten Pulvers, und die Ausbeute betrug dementsprechend 25-30% der theoretischen Ausbeute, bezogen auf das Material, das diesem zweiten Dialyseschritt unterworfen wurde.

### Isolierung von Fraktionen aus dem Produkt des zweiten Dialyseschrittes

Das Produkt des zweiten Dialyseschrittes wurde der Gelfiltration unter Verwendung von Sephadex G-25 SF unterworfen. Hiezu verwendete man eine Säule eines Durchmessers von 3,6 cm und einer Länge von 90 cm. Als Laufmittel wurde deionisiertes Wasser verwendet, und der Durchfluss des Laufmittels betrug 20 ml/Std. Es wurden Fraktionen von 10 ml aufgefangen.

Man erhielt dabei drei wirkstoffhaltige Fraktionen, die mit IIIa, IIIb und IIIc bezeichnet werden.

Die Fraktion IIIb wurde einer weiteren Gelfiltration unter Verwendung einer mit Sephadex G-25 SF gefüllten Säule unterworfen, und bei dieser zweiten Gelfiltration wurden 5 Fraktionen gewonnen, von denen die Fraktion III-b4 leicht gelblich war und die Fraktion b5 80% des gesamten Feststoffs enthielt, die durch Eindampfen jeder der fünf Fraktionen zur Trockene erhalten wurden.

Die Fraktion III-b5 wurde einer Hochdruckflüssigchromatographie unterworfen, wobei man zwei Hauptfraktionen erhielt, die mit b51 und b52 bezeichnet wurden. Diese Fraktionen wiesen im UV-Bereich eine Absorption bei 280 mn auf, und sie enthielten 40% der Feststoffe, die der Hochdruckflüssigchromatographie unterworfen worden waren.

### Beispiel 2

Es wurde nach dem Verfahren gemäss Beispiel 1 gearbeitet, wobei wiederum die erste Dialyse unter Verwendung einer Membran durchgeführt wurde, die Substanzen eines Molekulargewichts von mindestens 1000 Dalton zurückhielt. In diesem Fall wurden 115 g des gelblich-weissen Pulvers in 500 ml Wasser gelöst, und die Dialyse wurde gegen ein Volumen von 10 l Wasser durchgeführt. Die Dialyse wurde während 48 Stunden in der Kälte durchgeführt, wobei die Aussenlösung viermal gewechselt und verworfen wurde.

Die vereinigten Innenlösungen wurden lyophilisiert und man erhielt dabei 16 g Trockenprodukt. Dementsprechend betrug die Ausbeute, bezogen auf den der Dialyse unterworfenen Feststoff 14 Gew.-%.

Der zweite Dialyseschritt wurde in diesem Fall unter Verwendung einer Spektrapormembran durchgeführt, die Materialien eines Molekulargewichts von 3500 Dalton und mehr zurückhielt.

16 g des Produktes des ersten Dialyseschrittes wurden in 40 ml Wasser gelöst und gegen 1 l Wasser während 24 Stunden dialysiert. Dann wurde die Aussenlösung entfernt und aufbewahrt, und es wurde während weiterer 24 Stunden gegen 1 l frisches Wasser dialysiert.

Insgesamt wurde diese Dialyse unter täglichem Wechsel der Aussenlösung während neun Tagen durchgeführt, und nach dem neunten Tag wurde der Inhalt der Schläuche verworfen. Der Feststoffgehalt der zur Trockenen eingedampften Aussenlösungen war wie folgt:

| Aussenlösung, gewonnen nach dem Tag | Trockengewicht des Eindampfungsrückstandes in mg |
|---|---|
| 1 | 600 |
| 2 | 500 |
| 3 | 250 |
| 4 + 5 | 400 |
| 6 + 7 | 200 |
| 8 + 9 | 200 |

Insgesamt wurden also nach dieser neuntägigen Dialyse 2'150 mg gewonnen, und diese Trockenrückstände der Aussenlösungen wurden miteinander vereinigt und der weiteren Gelfiltration unterworfen.

2 g des Produktes des zweiten Dialyseschrittes wurden auf eine mit Sephadex G-25 SF gefüllte Säule eines Durchmessers von 2,6 cm und einer Länge von 90 cm aufgetragen.

Bei dieser Gelfiltration wurden die in Beispiel 1 beschriebenen Bedingungen aufrechterhalten, und es wurden wieder drei Fraktionen gewonnen, die nach dem Eindampfen zur Trockene die folgenden Feststoffgehalte aufwiesen:
IIIa : 45 mg
IIIb : 39 mg
IIIc : 22 mg.

Die Fraktion III-b wurde unter Verwendung einer mit Sephadex G-25 SF gefüllten Säule in fünf Wirkstoff-Fraktionen aufgeteilt, die mit III-b1, b2, b3, b4 und b5 bezeichnet werden. Es zeigte sich, dass die Fraktion b5 80% der Feststoffe sämtlicher Fraktionen b1 bis b5 enthielt.

Diese Fraktion III-b5 wurde durch Hochdruckflüssigchromatographie weiter gereinigt, wobei zwei Fraktionen gewonnen wurden, die im UV-Bereich eine Lichtabsorption bei 280 nm aufwiesen. Diese beiden Fraktionen werden mit b51 und b52 bezeichnet.

### Beispiel 3

Durch Strukturaufklärung mit der Infrarotspektrometrie und kernmagnetischen Resonanz konnte gezeigt werden, dass die gemäss Beispiel 1 und gemäss Beispiel 2 gewonnenen Fraktionen mit der Bezeichnung b51 und b52 dasjenige Glycosid der weiter vorne angegebenen Formel I enthielten, in welcher R Wasserstoff ist und das ein Molekulargewicht von 1001 Dalton aufweist.

Es konnte gezeigt werden, dass sich aus der reinen Fraktion b51 in Wasser allmählich eine Mischung aus b51 und b52 bildet, und dass andererseits die reine Fraktion b52 in Wasser ebenfalls eine Mischung aus b51 und b52 mit gleichem Mischungsverhältnis bildet. Das Gleichgewicht in der wässrigen Lösung liegt bei etwa 40 Gew.-% b52 und 60 Gew.-% b51. Aus der Gleichgewichtsmischung lässt sich wieder durch Säulenchromatographie eine Fraktion b51 und eine Fraktion b52 isolieren.

Weitere Untersuchungen zeigten, dass das Produkt b51 und das Produkt b52 stereoisomere Formen bezüglich des Kohlenstoffatomes in der 3-Stellung des heterocyclischen Kernes des Molekülteils der 2-Oxindol-3-essigsäure darstellen.

### Beispiel 4

Gemäss Beispiel 1 und Beispiel 2 wurden durch die Gelfiltration auf der mit Sephadex G-25 gefüllten Säule drei Fraktionen mit der Bezeichnung IIIa, IIIb und IIIc isoliert.

In diesem Beispiel wird jetzt die Fraktion IIIa einer weiteren Trennung unter Verwendung einer mit Sephadex G-25 gefüllten Säule unterworfen. Auf diese Säule wurden 140 mg der Fraktion IIIa aufgetragen und bei dieser Gelfiltration erhielt man eine Fraktion mit der Bezeichnung A3, die nach dem Eindampfen 30 mg Feststoff lieferte und eine Fraktion mit der Bezeichnung A4, die nach dem Eindampfen zur Trockene 66 mg lieferte.

Die Gesamtmenge, die in den Fraktionen A3 und A4 enthalten war, entsprach also einer Ausbeute von 65% des aufgetragenen Feststoffes.

Die Fraktion A3 wurde einer Hochdruckflüssigchromatographie unterworfen und dabei wurden zwei Hauptfraktionen mit der Bezeichnung A41 und A42 gewonnen.

Die Wirkstoffe dieser beiden Fraktionen besassen ein Molekulargewicht von 1163 Dalton, und das Massenspektrum der Fraktion A41 war identisch mit demjenigen der Fraktion A42.

Das Massenspektrum dieser Fraktionen A41 und A42 war also um 162 Dalton höher als dasjenige der gemäss Beispiel 3 isolierten Fraktionen b51 und b52, woraus geschlossen werden kann, dass sich das gemäss Beipiel 3 isolierte Wirkstoffpaar b51/b52 von dem jetzt isolierten Wirkstoffpaar A41/A42 durch eine glycosidisch gebundene Hexoseeinheit unterscheidet.

Durch weitere analytische Untersuchungen konnte ferner bewiesen werden, dass das durch saure Hydrolyse gebildete Aglycon des Paares A41/A42 identisch mit dem Aglycon des Paares b51/bS2 ist, und durch kernmagnetische Resonanzspektren konnte ferner bewiesen werden, dass das Glycosidenpaar A41/A42 der Formel I entspricht, wobei R der Rest einer Glucoseeinheit ist.

Auch beim Glycosidenpaar A41/A42 wandeln sich in wässriger Lösung die Glycoside dieses Paars ineinander um, wobei sich auch hier, unabhängig davon, ob man vom Glycosid A41 oder vom Glycosid A42 ausgeht, einige Zeit ein identisches Gleichgewichtsgemisch 1 einstellt. Auch in diesem Fall besteht der Unterschied zwischen dem Glycosid A41 und dem Glycosid A42 in der sterischen Konfiguration am chiralen Zentrum in der 3-Stellung des Indolkerns der 2-Oxindol-3-essigsäure.

### Beispiel 5

Das gemäss Beispiel 2 isolierte Glycosidenpaar b51/b52 wurde unter milden alkalischen Bedingungen behandelt. Dabei konnte ein Glycosid der folgenden Struktur 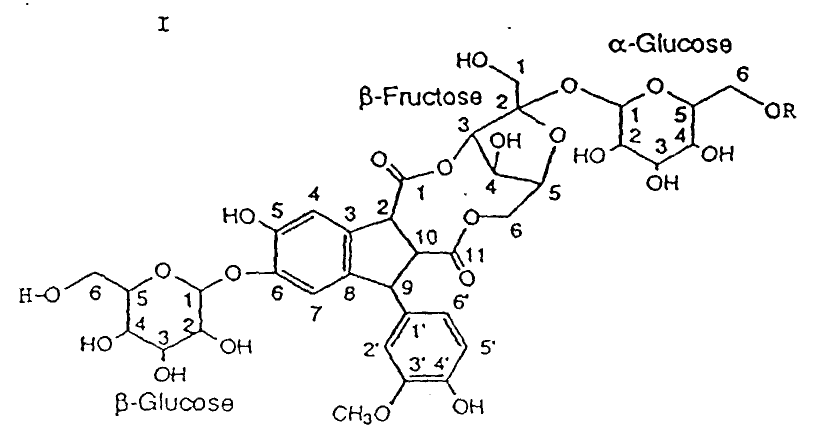 isoliert werden, wobei in diesem Glycosid R ein Wasserstoffatom und Z ebenfalls ein Wasserstoff war.

### Beispiel 6

In diesem Beispiel wurde das gemäss Beispiel 4 erhaltene Glycosidenpaar A41/A42 der Behandlung mit der β-Glucosidase unter alkalischen Bedingungen unterworfen.

In diesem Fall erhielt man ein Glycosid, welches der im Beispiel 5 angegebenen Struktur entspricht, wobei jedoch jetzt der Rest R ein glycosidisch gebundener Glucoserest war.

### Beispiel 7

3 bis 5 mg des gemäss Beispiel 3 isolierten Glycosidenpaares b51/b52 wurden in 250-330 ul einer 1,0 normalen Salzsäure gelöst. Man behandelte die Lösung sechs Stunden lang unter Vakuum bei 100° C.

Anschliessend wurde getrocknet, mit Wasser gewaschen und zentrifugiert, wobei ein dunkelbrauner Niederschlag erhalten wurde. Dieser Niederschlag wurde verworfen, und die darüberstehende Flüssigkeit durch Hochdruckflüssigchromatographie gereinigt. Durch dieses Reinigungsverfahren wurde das Aglycon der durch die folgende Formel II 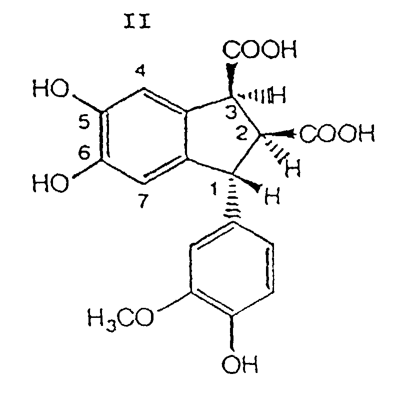 veranschaulichten relativen Konfiguration isoliert.

Die Struktur dieser Indan-dicarbonsäure wurde durch Massenspektren und kernmagnetische Resonanzspektren bewiesen.

In gleicher Weise wurde auch das gemäss Beispiel 4 hergestellte Glycosidenpaar A41/A42 der sauren Hydrolyse mit der 1-normalen Salzsäure unterworfen.

Auch in diesem Falle wurde das wasserlösliche Material der Hochdruckflüssigchromatographie unterworfen und man konnte wieder als Aglycon die Verbindung der oben angegebenen Formel II isolieren.

### Beispiel 8

### Testung der pharmakologischen Wirksamkeit der Glycoside der Formel I

Zur Durchführung des pharmakologischen Testes wurden weibliche Mäuse verwendet, die 5-7 Wochen alt waren und ein Gewicht von 19-22 g aufwiesen.

Am Tage 0 wurden an jedes Tier 1,5 x 10⁵ Tumor S180 Zellen durch intraperitoneale Injektion verabreicht.

Am Tage 1 wurden die Tiere nach dem Zufallsprinzip in einzelne Gruppen sortiert, wobei jede Gruppe 6 Mäuse umfasste und in jeder Gruppe die Tiere ein ähnliches Gewicht aufwiesen.

Nach einer Zeit von einer Woche hatten sich bei sämtlichen Tieren sämtlicher Gruppen deutlich sichtbare Tumore ausgebildet.

In der Gruppe zu Vergleichszwecken (Gruppe 1) wurden die Tiere keiner weiteren Behandlung unterworfen. An die Tiere der Gruppen 2-6 wurde anschliessend durch intraperitoneale Injektion das Glycosid der Formel I verabreicht, in welchem R ein Wasserstoffatom war. Dieses Glycosid wurde am Tage 1 in einer Dosierung von 50 µg pro Maus (Gruppe 2), bzw. 75 µg pro Maus (Gruppe 3), bzw. 100 µg pro Maus (Gruppe 4), bzw. 150 µg pro Maus (Gruppe 5), bzw. 200 µg pro Maus (Gruppe 6) verabreicht. Die Injektionen wurden in der selben Dosis an den Tagen 3, 5, 7 und 9 wiederholt.

Dabei zeigte es sich, dass auch bei der höchsten angewandten Dosierung in der Gruppe 6 keine toxische Wirkung auf die Tiere nachgewiesen werden konnte.

Zehn bis zwölf Tage nach Beginn des Versuches waren sämtliche Tiere der Vergleichsgruppe (Gruppe 1) infolge des Tumors verstorben. Bei den mit dem Glycosid behandelten Mäusen wurde jedoch eine Heilung bei 50% der Tiere erreicht. Diese Tiere lebten bei Abbruch des Versuches bereits mehr als 50 Tage bei guter Gesundheit. Bei den restlichen Tieren der Gruppen 2-6 wurde eine deutliche Rückbildung der Tumore beobachtet.

Die Versuchsergebnisse waren bei verschiedenen anderen menschlichen Tumorzellen ähnlich, wie dies oben für die Tumor S-180-Zellen beschrieben ist. Auch in diesem Fall konnte mit dem Glycosid der Formel I rasch ein Ausheilen der Tumore erreicht werden.

Des weiteren wurde das Glycosid auch in Zellkulturen auf seine Tumoraktivität untersucht. Dabei zeigte es sich, dass dieses Glycosid keine cytotoxischen Eigenschaften auf die Tumorzellen ausübte.

Diese völlig überraschende Ausheilung der Tumore durch die Verabreichung der aus den Pollenextrakten isolierten Naturstoffe ist deshalb auf eine Modulierung der Immunreaktion der lebenden Tiere und nicht auf cytotoxische Eigenschaften der aus dem Naturstoff isolierten Glycoside zurückzuführen.

### Beispiel 9

Analoge Tests, wie im Beispiel 8 beschrieben, wurden mit dem Glycosid der Formel I durchgeführt, in welchem R ein weiterer glycosidisch gebundener Glucoserest ist. In diesem Falle waren die Testergebnisse denjenigen des Beispiels 8 sehr ähnlich. Auch hier ist selbst bei der niedrigsten Dosierung von nur 50 µm pro Maus/Injektion in der ersten Testgruppe bei 30% der Tiere eine vollkommene Heilung festgestellt worden.

## Patentansprüche

1. Pharmakologisch wirksame Glycoside, dadurch gekennzeichnet, dass sie die folgende Formel 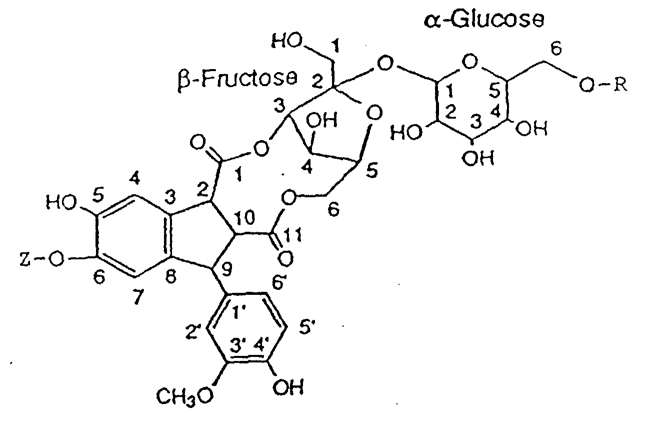 aufweisen, wobei in dieser Formel
R ein Wasserstoffatom oder ein glycosidisch gebundener Hexoserest oder eine Gruppe aus 2 oder mehr Hexoseresten ist und
Z ein Wasserstoffatom oder eine Gruppe der Formel A 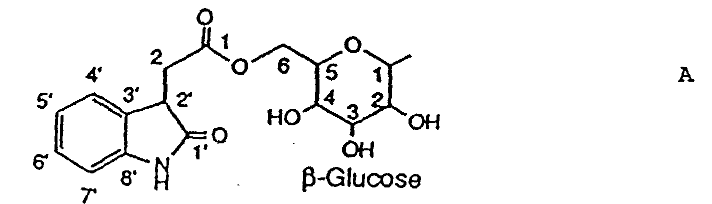 oder der Formel B 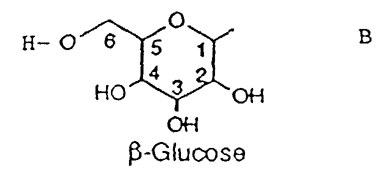 bedeutet,
oder dass diese Glycoside die folgende Formel 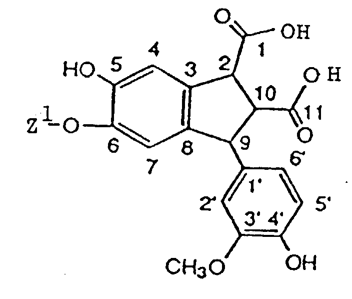 aufweisen, wobei in diesen Glycosiden
Z¹ ein glycosidischer Rest der oben angegebenen Formel A oder der oben angegebenen Formel B ist.

2. Glycosid gemäss Anspruch 1, dadurch gekennzeichnet, dass es die folgende Formel I 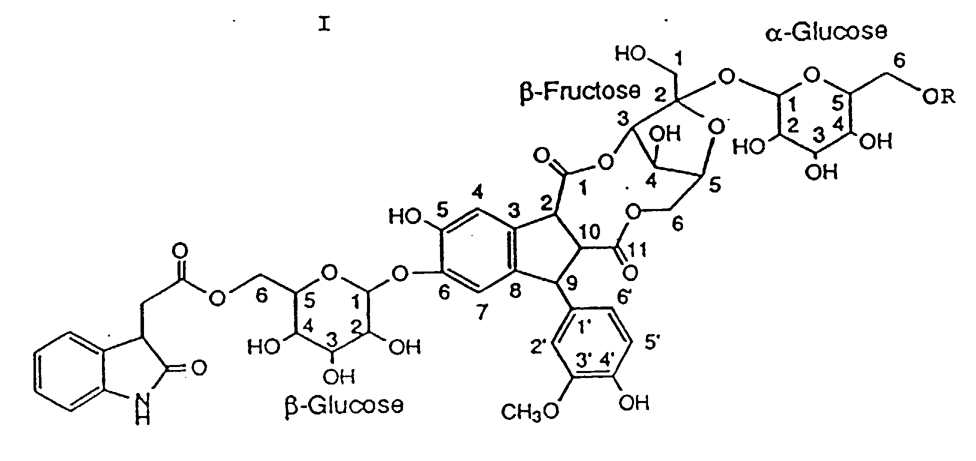 aufweist, wobei in dieser Formel
R ein Wasserstoffatom oder ein Hexoserest, vorzugsweise ein Glucoserest ist.

3. Indanderivate mit pharmakologischer Aktivität, dadurch gekennezeichnet, dass sie die folgende Formel III 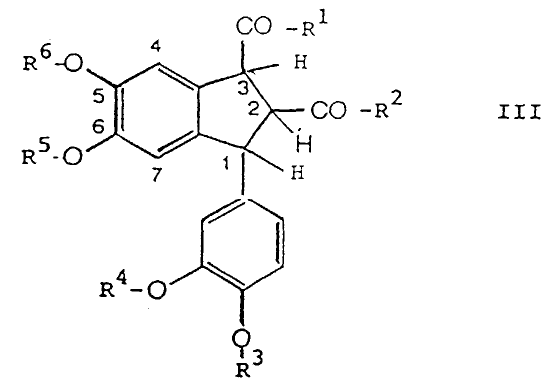 aufweisen, wobei in dieser Formel
R¹ und R² unabhängig voneinander Gruppen der Formel
-O-X,
in welchen
X für Wasserstoff, substituierte oder unsubstituierte Alkylgruppen mit 1-6 Kohlenstoffatomen, beispielsweise Aralkylgruppen oder substituierte oder unsubstituierte Arylgruppen oder ein oder mehrere esterartig gebundene Hexosereste stehen oder
R¹ und R² gemeinsam eine zweiwertige Gruppierung der Formel
-O- oder -O-Y-O-
bedeutet, wobei in diesen Formeln
Y für einen zweiwertigen organischen Rest, insbesondere einen aliphatischen, cycloaliphatischen oder aromatischen Kohlenwasserstoffrest steht und
R³, R⁴, R⁵ und R⁶ unabhängig voneinander für Wasserstoffatome, gegebenenfalls substituierte Alkylreste, gegebenenfalls substituierte Cycloalkylreste, gegebenenfalls substituierte Arylreste oder Acylreste von aliphatischen, cyclo-aliphatischen oder aromatischen Carbonsäuren oder ein oder mehrere Hexosereste stehen.

4. Indanderivate gemäss Anspruch 3, dadurch gekennzeichnet, dass in ihnen
R¹ und R² für eine Hydroxygruppe steht,
R³, R⁴ und R⁵ ein Wasserstoffatom ist und R⁴ für einen Methylrest steht.

5. Indanderivate gemäss Anspruch 3 oder 4, dadurch gekennzeichnet, dass sie die durch die folgende Formel IIIa 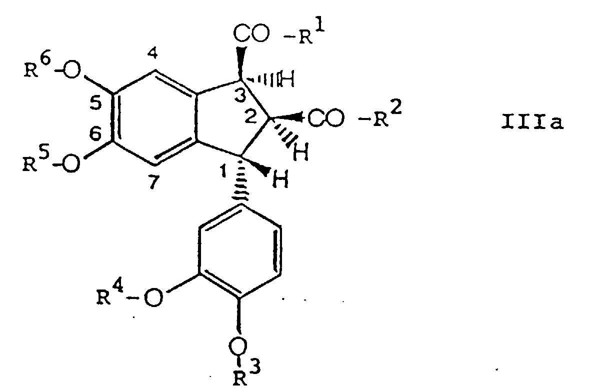 veranschaulichte relative Konfiguration aufweisen, wobei in dieser Formel IIIa
R¹, R², R³, R⁴, R⁵ und R⁶ die gleiche Bedeutung aufweisen, wie in Formel III.

6. Indanderivate gemäss Anspruch 5, dadurch gekennzeichnet, dass sie die durch die folgende Formel IIa 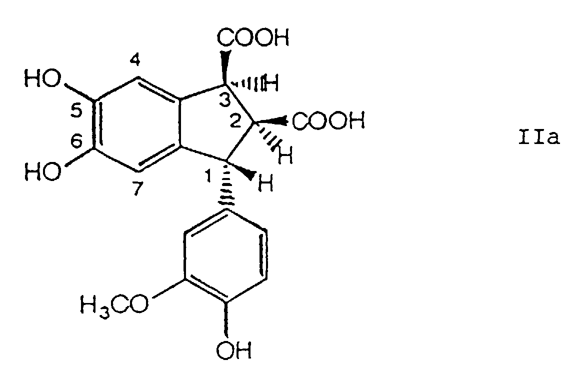 veranschaulichte relative Konfiguration aufweisen.

7. Pharmazeutisches Präparat zur Modulierung des Immunsystems warmblütiger Tiere, dadurch gekennzeichnet, dass es als Wirkstoff ein Glycosid der in Anspruch 1 angeführten Strukturformel oder ein Indanderivat der Formel III gemäss Anspruch 3 enthält.

8. Pharmazeutisches Präparat gemäss Anspruch 7, dadurch gekennzeichnet, dass es als Wirkstoff ein Glycosid gemäss Anspruch 2 oder ein Indanderivat gemäss einem der Ansprüche 4 bis 6 enthält.

9. Pharmazeutisches Präparat gemäss Anspruch 7 oder 8, dadurch gekennzeichnet, dass es ein Präparat zur in vivo Bekämpfung von Tumoren oder zur Bekämpfung von Viruserkrankungen, insbesondere durch Retroviren verursachter Krankheiten, beispielsweise Aids, ist und dass es die Wirkstoffe der angegebenen Formeln oder pharmazeutisch annehmbare Salze dieser Wirkstoffe enthält.

10. Verfahren zur Gewinnung der Glycoside gemäss Anspruch 1, dadurch gekennzeichnet, dass man aus dem wässrigen Extrakt von Pflanzenpollen diejenigen Glycoside der folgenden Formel gewinnt, 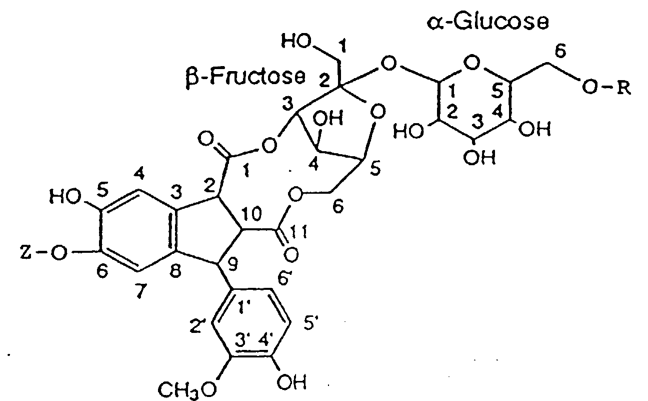 in welchen
R ein Wasserstoffatom oder ein glycosidische gebundener Hexoserest ist und
Z für den über die β-Glucose-Einheit gebundenen glycosidischen Rest der Formel A 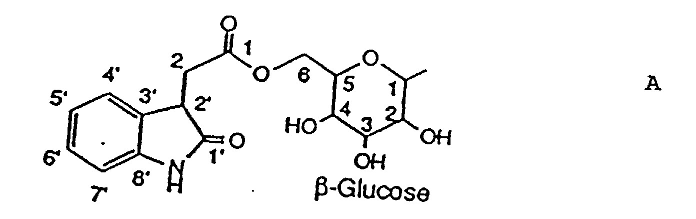 steht, indem man aus dem wässrigen Extrakt der Pflanzenpollen durch Dialyse unter Verwendung einer Membran, die Substanzen eines Molekulargewichts von über 1000 Dalton zurückhält, diejenigen Bestandteile entfernt, die diese Membran passieren, und dann einen zweiten Dialyseschritt unter Verwendung einer Membran durchführt, die Materialien eines Molekulargewichts von über 3500 Dalton, vorzugsweise über 2000 Dalton, zurückhält, wobei die Glycoside aus dem die Membran passierenden Dialysat durch eine Hochdruckflüssigchromatographie isoliert werden,
und dann gegebenenfalls die so erhaltenen Produkte unter leicht basischen Bedingungen behandelt, wobei man diejenigen Glycoside der oben angegebenen Formel erhält, in welcher Z die β-Glucoseeinheit der Formel B ist, oder
dass man das Glicosid der angegebenen Formel, in welchem Z für die Gruppe der oben angegebenen Formel A steht, einer Esterabspaltung unterwirft, wobei man dasjenige Glycosid der Formel 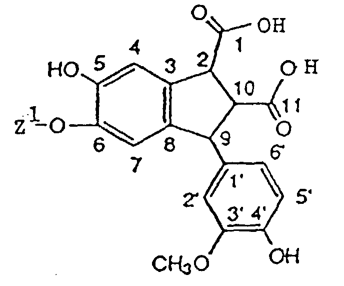
isoliert, in welchem Z¹ für eine glycosidische Gruppe der Formel A oder den Glucoserest der Formel B steht.

11. Verfahren zur Herstellung des Aglycons der Formel III gemäss Anspruch 3,
in welchem
R¹ und R² für Hydroxygruppen stehen,
R³, R⁵ und R⁶ Wasserstoff bedeuten und
R⁴ eine Methylgruppe ist,
dadurch gekennzeichnet, dass man die nach dem Verfahren gemäss Anspruch 10 hergestellten Glycoside einer sauren hydrolytischen Spaltung unterwirft und das Aglycon der Formel III isoliert.

12. Verfahren nach Anspruch 11, dadurch gekennzeichnet, dass man aus dem Produkt der hydrolytischen Spaltung ein Aglycon der Formel IIa gemäss Anspruch 6 isoliert.

13. Verfahren gemäss Anspruch 11 oder 12, dadurch gekennzeichnet, dass man aus dem Aglycon der Formel III, bzw. IIa, diejenige Verbindung der Formel III gemäss Anspruch 3, bzw. der Formel IIIa gemäss Anspruch 5 durch Verätherungsreaktionen, Veresterungsreaktionen oder Aetherspaltung synthetisch herstellt, in welchen mindestens einer der Substituenten R¹ und R² eine andere Bedeutung aufweist als diejenige von Hydroxygruppen, oder mindestens einer der Reste R³, R⁵ oder R⁶ eine andere Bedeutung aufweist als diejenige eines Wasserstoffatoms oder der Rest R⁴ eine andere Bedeutung aufweist als diejenige eines Methylrestes.

## Claims

1. Pharmacologically active glucosides, characterized in that they have the following formula 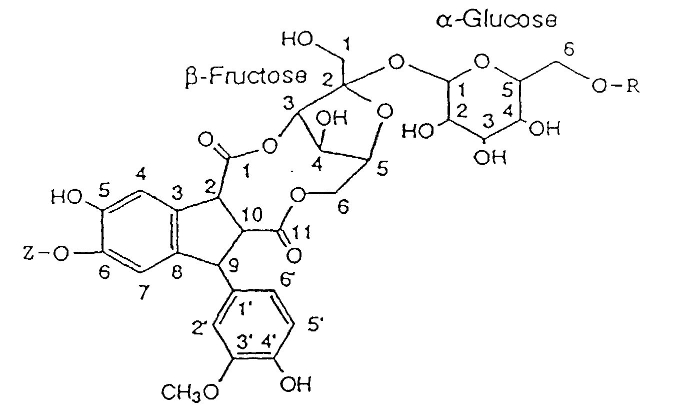 whereby in said formula
R is a hydrogen atom or a hexose residue bonded in a glucosidical linkage or a group of two or more hexose residues and
Z is a hydrogen atom or a group of the formula A 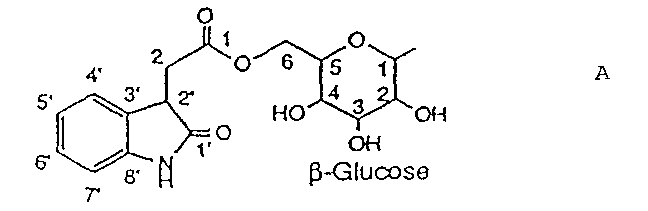 or of the formula B 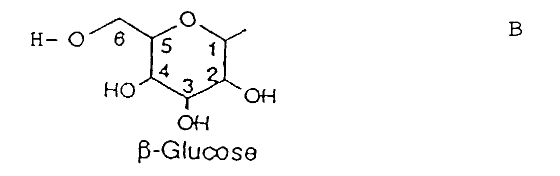 or that said glucosides have the following formula 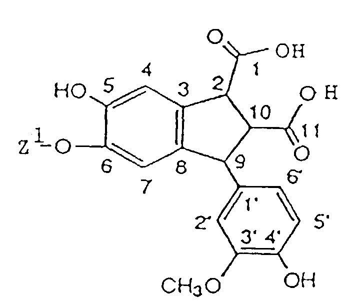 whereby
Z¹ represents a glycosidical residue of the above indicated formula A or of the above indicated formula B.

2. A glucoside according claim 1, characterized in that it has the following formula I 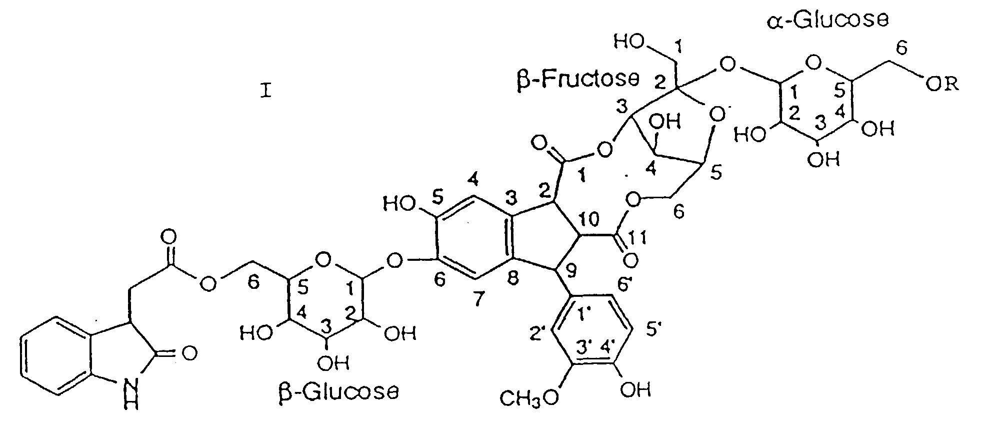 whereby in said formula
R is a hydrogen atom or a hexose residue, preferably a glucose residue.

3. Indan derivatives having a pharmacological activity, characterized in that they have the following formula III 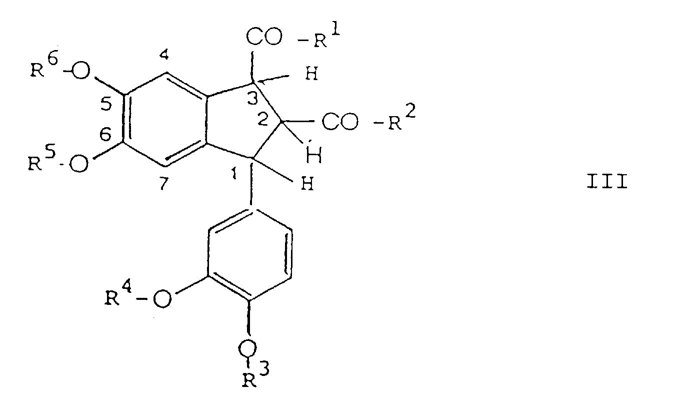 whereby in said formula
R¹ and R² represent independently from each other the formula
-O-X,
wherein
X represents hydrogen, substituted or unsubstituted alkyl groups having 1-6 carbon atoms, for example aralkyl groups or substituted or unsubstituted aryl groups or one or more hexose residues bonded in an ester-linkage or R¹ and R² represent both a bivalent group of the formula
-O- or -O-Y-O-
whereby in these formulas
Y represents a bivalent organic residue, in particular an aliphatic, cycloaliphatic or aromatic hydrocarbon residue and
R³, R⁴, R⁵ and R⁶ represent independently from each other hydrogen atoms, optionally substituted alkyl residues, optionally substituted cycloalkyl residues, optionally substituted aryl residues or acyl residues of aliphatic, cycloaliphatic or aromatic carboxylic acids or one or more hexose residues.

4. Indan derivatives according to claim 3, characterized in that
R¹ and R² represent a hydroxy group,
R³, R⁴ and R⁵ represent a hydrogen atom and R⁴ represents a methyl residue.

5. Indan derivatives according to claim 3 or 4, characterized in that they display the relative configuration illustrated by the following formula IIIa 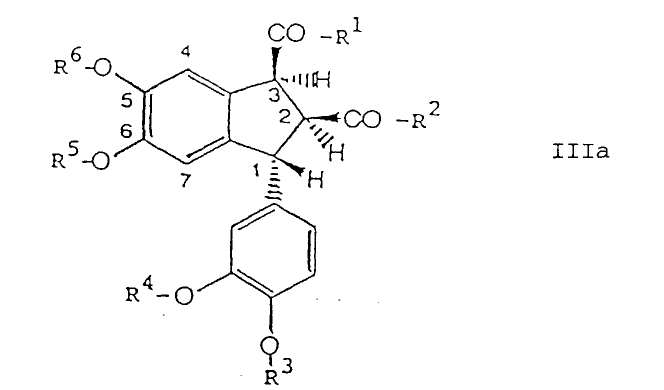 wherein in said formula IIIa
R¹, R², R³, R⁴, R⁵ and R⁶ have the same meaning as in formula III.

6. Indan derivatives according to claim 5, characterized in that they display the relative configuration illustrated by the following formula IIa

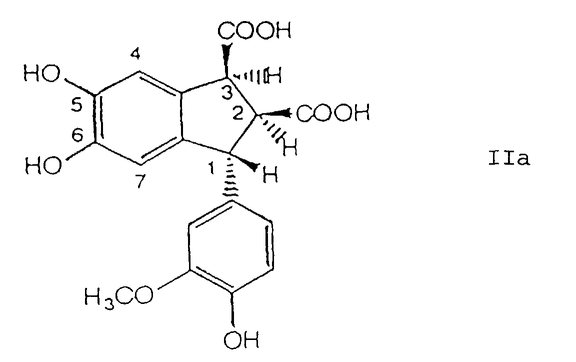

7. Pharmaceutical composition for the modulation of the immune system of warm blooded animals, characterized in that it contains a glucoside of the structural formula according to claim 1, or an indan derivative of the formula III according to claim 3 as active component.

8. Pharmaceutical composition according to claim 7, characterized in that it contains a glucoside according to claim 2 or an indan derivative according to one of the claims 4 to 6.

9. Pharmaceutical composition according to claim 7 or 8, characterized in that it contains a composition for the *in vivo* treatment of tumors or for the treatment of virus diseases, particularly retrovirus related diseases, for example AIDS, and that it contains the active components of the indicated formula or pharmaceutical acceptable salts of said active components.

10. Process for the isolation of glucosides according to claim 1, characterized in that said glucosides of the following formula 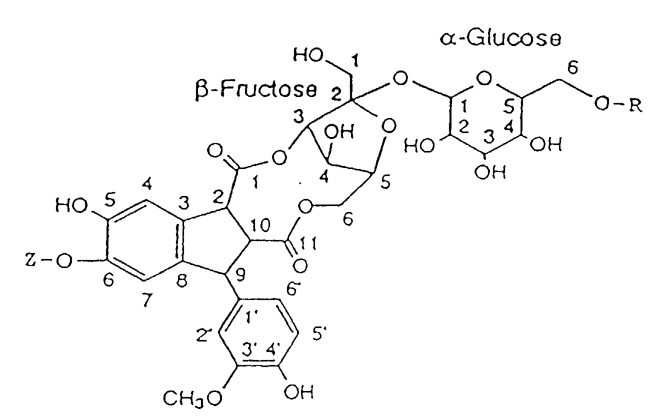 are extracted from an aqueous extract of vegetable pollen
wherein
R is a hydrogen atom or a hexose residue bonded in a glycosidical linkage, and
Z represents a glycosidical residue of the formula A 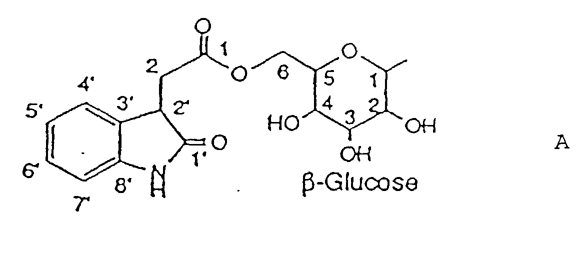
bonded through the β-glucose unit, by removing through dialysis from the aqueous extract of the vegetable pollen those agents that are diffusing through said membrane, by using a membrane that retains substances having a molecular weight greater than 1000 Daltons, and then through a second dialysis step through the use of a membrane retaining materials of a molecular weight greater than 3500 Daltons, preferably greater than 2000 Daltons, whereby the glucosides from the dialysate diffusing through the membrane are isolated through high pressure liquid chromatography,
and then optionally the products thus obtained are treated under slightly alkaline conditions, whereby those glucosides of the above indicated formulas are obtained, in which Z represents the β-glucose moiety of the formula B, or
that the glucoside of the indicated formula, in which Z represents the group of the above indicated formula A subjected to an ester cleavage, whereby the one glucoside of the formula 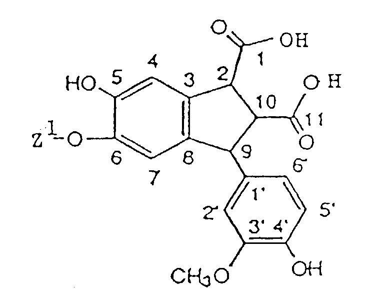 is isolated, in which Z¹ represents a glycosidical group of the formula A or a glucose residue of the formula B.

11. Process for the production of the aglycon of the formula III according to claim 3,
whereby
R¹ and R² represent hydroxy groups,
R³, R⁵ and R⁶ represent hydrogen and
R⁴ is a methyl group,
characterized in that the glucosides prepared according to the method of claim 10 are subjected to an acidic hydrolytic cleavage and the aglycon of the formula III is isolated.

12. Method according to claim 11, characterized in that an aglycon of the formula IIa according to claim 6 is isolated from the product of the hydrolytic cleavage.

13. Method according to claim 11 or 12, characterized in that from the aglycon of the formula III or IIa, respectively, those compounds of the formula III according to claim 3, or of the formula IIIa according to claim 5 are prepared synthetically through etherification reactions, esterification reactions or ether cleavages, whereby at least one of the substituents R¹ and R² has a different meaning than the one of hydroxy groups, or at least one of the residues R³, R⁵ or R⁶ have a different meaning than the one of a hydrogen atom or at the residue R⁴ displays a different meaning than the one of a methyl residue.

## Revendications

1. Glucosides activité pharmacologique, caractérisés en ce qu'ils présentent la formule suivante 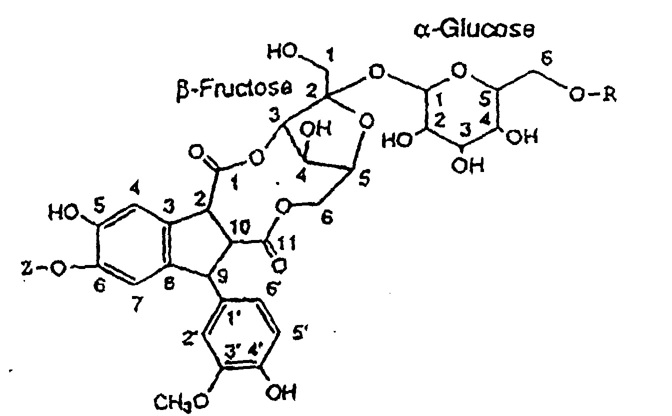 dans laquelle
R représente un atome d'hydrogène ou un reste hexose à liaison glucosidique ou un groupe de 2 ou plus de deux restes hexose et
Z représente un groupe de la formule A 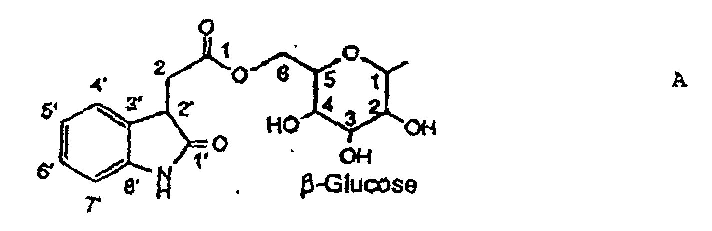 ou de la formule B 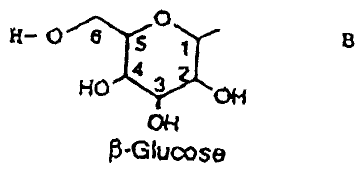 ou en ce que ces glucosides présentent la formule suivante 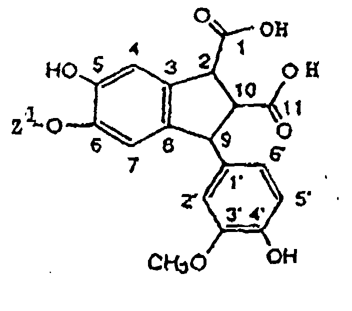 où, dans ces glucosides,
Z¹ représente un reste glucosidique de la formule A ci-avant ou de la formule B ci-avant.

2. Glucoside selon la revendication 1, caractérisé en ce qu'il présente la formule I suivante 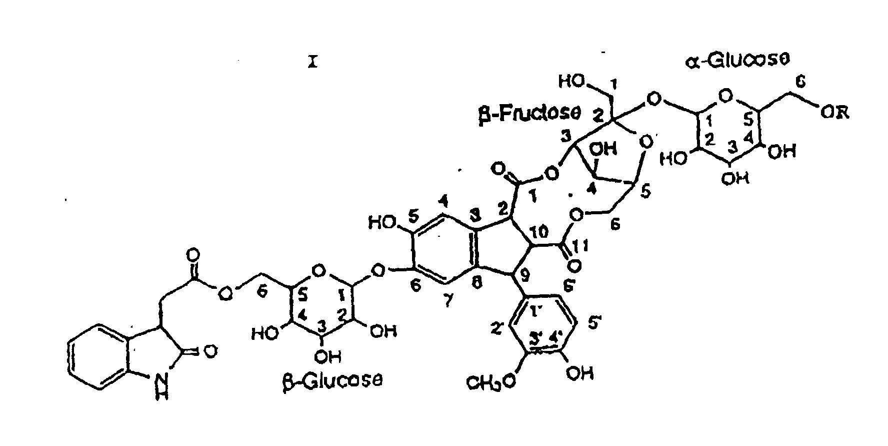 dans laquelle
R représente un atome d'hydrogène ou un reste hexose, de préférence un reste glucose.

3. Dérivés d'indane à activité pharmacologique, caractérisés en ce qu'il présentent la formule III suivante 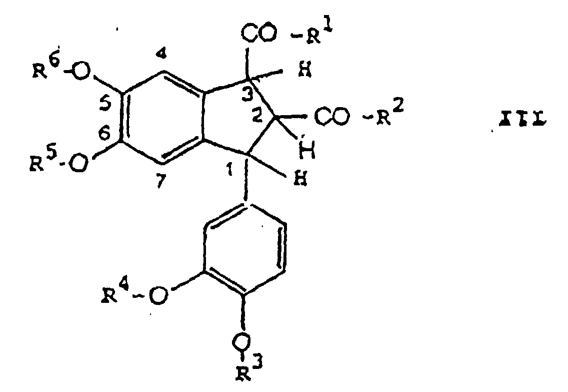 dans laquelle
R¹ et R² représentent indépendamment l'un de l'autre des groupes de formule
-O-X,
dans laquelle
X représente l'hydrogène, des radicaux alkyles substitués ou non substitué, comportant de 1 à 6 atomes de carbone, par exemple des radicaux aralkyle, ou des radicaux aryle substitués ou non substitués, ou un ou plusieurs restes hexose à liaison ester, ou bien
R¹ et R² représentent ensemble un groupement bivalent de formule
-O- ou -O-Y-O-
dans laquelle
Y représente un reste organique bivalent, en particulier un reste hydrocarbure aliphatique, cycloaliphatique ou aromatique, et
R³, R⁴, R⁵ et R⁶ représentent indépendamment les uns des autres un atome d'hydrogène, un radical alkyle éventuellement substitué, un radical cycloalkyle éventuellement substitué, un radical aryle éventuellement substitué ou des restes acyle d'acides carboxyliques aliphatiques, cycloaliphatiques ou aromatiques, ou un ou plusieurs restes hexose.

4. Dérivés d'indane selon la revendication 3, caractérisés en ce que, dans ces dérivés,
R¹ et R² représentent un radical hydroxy,
R³, R⁴ et R⁵ représentent un atome d'hydrogène et R⁴ représente un radical méthyle.

5. Dérivés d'indane selon la revendication 3 ou 4, caractérisés en ce qu'ils présentent la configuration relative illustrée par la formule Illa suivante 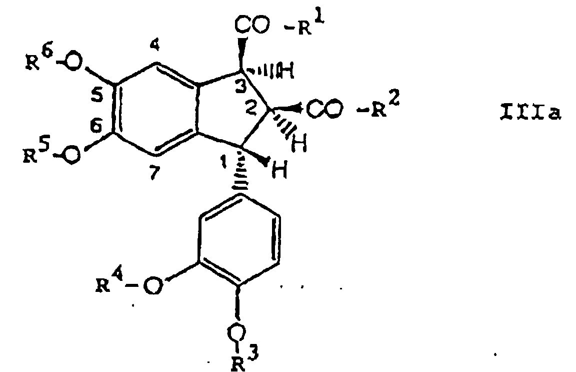 dans laquelle
R¹, R², R³, R⁴, R⁵ et R⁶ ont les mêmes significations que dans la formule IIIa.

6. Dérivés d'indane selon la revendication 5, caractérisés en ce qu'ils présentent la configuration relative illustrée par la formule IIa suivante

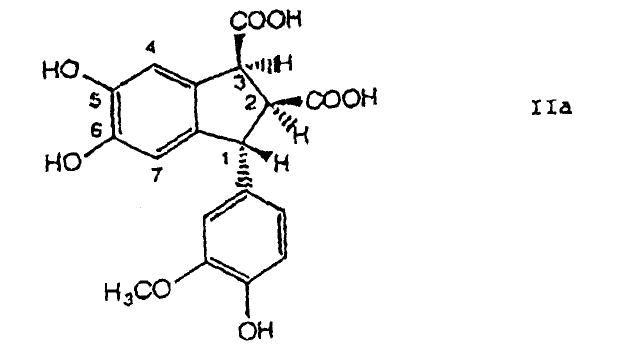

7. Préparation pharmaceutique pour la modulation du système immunitaire d'animaux à sang chaud, caractérisée en ce qu'elle contient en tant qu'agent actif un glucoside de la formule structurelle indiquée à la revendication 1 ou un dérivé d'indane de la formule III selon la revendication 3.

8. Préparation pharmaceutique selon la revendication 7, caractérisée en ce qu'elle contient en tant qu'agent actif un glucoside selon la revendication 2 ou un dérivé d'indane selon l'une des revendications 4 à 6.

9. Préparation pharmaceutique selon la revendication 7 ou 8, caractérisée en ce qu'elle consiste en une préparation destinée à combattre des tumeurs in vivo ou à combattre des maladies virales, en particulier des maladies provoquées par des rétrovirus, par exemple le SIDA, et qu'elle contient les agents actifs des formules indiquées ou des sels pharmaceutiquement acceptables de ces agents actifs.

10. Procédé d'obtention des glucosides selon la revendication 1, caractérisé en ce que l'on obtient à partir de l'extrait aqueux de pollens végétaux des glucosides de la formule suivante, 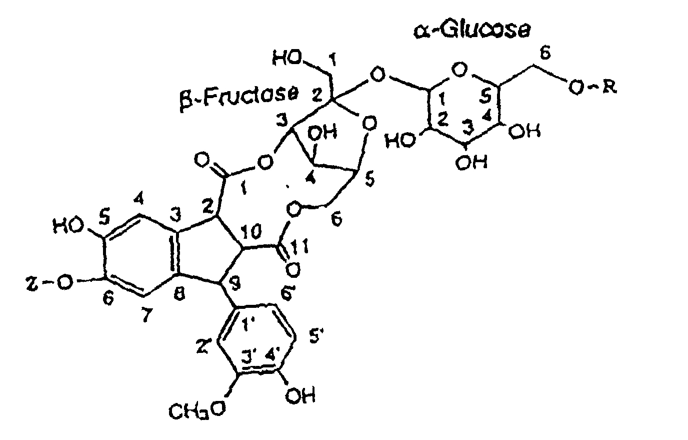 dans laquelle
R représente un atome d'hydrogène ou un reste hexose à liaison glucosidique et
Z représente le reste glucosidique, lié via l'unité de β-glucose, de la formule A 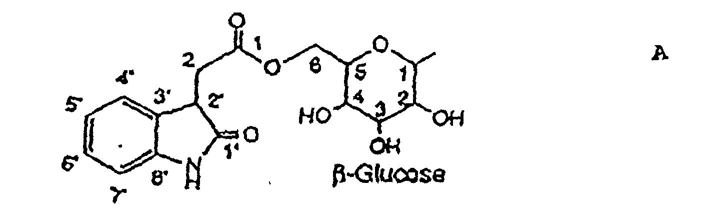 en que l'on retient de l'extrait aqueux du pollen végétal, par dialyse sur membrane, les substances d'un poids moléculaire supérieur à 1000 Dalton, que l'on élimine les composants qui traversent la membrane et qu'ensuite, dans une deuxième étape de dialyse sur membrane, on retient les matières d'un poids moléculaire supérieur à 3500 Dalton, de préférence supérieur à 2000 Dalton, en isolant les glucosides du dialysat traversant la membrane par chromatographie en phase liquide sous haute pression,
et que l'on traite éventuellement les produits ainsi obtenus dans des conditions légèrement basiques, obtenant ainsi les glucosides de la formule indiquée ci-dessus, dans laquelle Z représente l'unité de β-glucose de la formule B, ou bien
que l'on soumet le glucoside de la formule indiquée, dans lequel Z représente le groupe de la formule A ci-avant, à une fission estérique, isolant le glucoside respectif de la formule 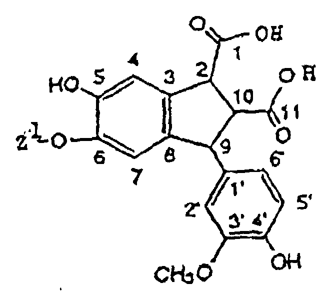 dans
laquelle Z¹ représente un groupe glucosidique de la formule A ou le reste glucose de la formule B.

11. Procédé de préparation de l'aglucone de la formule III selon la revendication 3,
dans laquelle
R¹ et R² représentent des radicaux hydroxy,
R³, R⁵ et R⁶ représentent l'hydrogène et
R⁴ représente un radical méthyle,
caractérisé en ce que l'on soumet le glucoside préparé par le procédé selon la revendication 10 à une fission hydrolytique acide et que l'on isole l'aglucone de la formule III.

12. Procédé selon la revendication 11, caractérisé en ce que l'on isole, à partir du produit de la fission hydrolytique, un aglucone de la formule IIa selon la revendication 6.

13. Procédé selon la revendication 11 ou 12, caractérisé en ce que l'on synthétise à partir de l'aglucone de la formule III, et/ou IIa, le composé de la formule III selon la revendication 3, et/ou de la formule IIIa selon la revendication 5, par des réactions d'éthérification, des réactions d'estérification ou fission éthérique, et que dans ledit composé au moins un des substituants R¹ et R² possède une signification autre que celle d'un radical hydroxy, ou bien au moins un des radicaux R³, R⁵ ou R⁶ possède une signification autre que celle d'un atome d'hydrogène ou que le radical R⁴ possède une signification autre que celle d'un radical méthyle.
